# EUROPEAN PATENT APPLICATION

(11) **EP 3 709 307 A1**
(43) Date of publication of application: **16.09.2020**
(21) Application number: 19162456.8
(22) Date of filing: 13.03.2019
(51) Int. Cl.: G16H 40/63, G16H 10/60, G16H 30/20

(54) **STORING AND RENDERING AUDIO AND/OR VISUAL CONTENT OF A USER**

(71) Applicant: Koninklijke Philips N.V., 5656 AG Eindhoven (NL)
(72) Inventor: TALGORN, Elise Claude Valentine, 5656 AE Eindhoven (NL); GEURTS, Lucas Jacobus Franciscus, 5656 AE Eindhoven (NL); TOSHNIWAL, Omna, 5656 AE Eindhoven (NL); PEKEL, Atike, 5656 AE Eindhoven (NL); BUIL, Vincentius Paulus, 5656 AE Eindhoven (NL)
(74) Representative: de Haan, Poul Erik

(57) **Abstract**

There is provided a system (100) for storing audio and/or visual content of a user. The system (100) comprises one or more processors (102) configured to, in response to a detection of a gesture and/or voice command of a user, identify a location on a body of a subject indicated by the gesture and/or voice command. The system (100) also comprises one or more processors (102) configured to control at least one memory (104) to store an indication of the identified location on the body of the subject with audio and/or visual content of the user captured for a time period after or during which the gesture and/or voice command of the user is detected.

## Description

### FIELD OF THE INVENTION

The disclosure relates to systems and methods for storing audio and/or visual content of a user and for rendering audio and/or visual content of a user.

### BACKGROUND OF THE INVENTION

It is often the case that a subject is provided with a vast amount of information during any given day and it can therefore be difficult for a subject to remember all of the information that they have been provided, let alone remember all of the information accurately. Sometimes the information can be important and it can be problematic if the subject forgets the information or recalls the information incorrectly. It can also be difficult for a subject to retrieve or filter desired information easily amongst the vast amount of information that may be available to them.

This is particularly the case in the healthcare domain, where patient engagement is important for the subject (e.g. a patient) to understand their condition, the progression of a disease, a recommended treatment, etc. Many explanations may be provided by a medical professional (e.g. a doctor) during a consultation and it is often the case that a subject will not be able to recall the explanations accurately at a later time. Also, as there is often vast amounts of information contained in medical images (including annotations and medical reports), information provided by way of medical images can be difficult for the subject to understand, especially when the information is looked at out of context, i.e. without the associated explanation from the medical professional.

A directory may be created containing all documents (e.g. medical images, reports, background brochures, anatomical representations, etc.) from consultations with a doctor for a subject to consult at home. However, this can be an overwhelming amount of information for the subject, which they would have to filter and link together. This can be complex and time-consuming for the subject, which means that they will not be engaged with the information and thus will be discouraged from consulting it. Also, a subject is required to report events (e.g. symptoms) associated with their health to a medical professional in between consultations. However, it can be difficult and tedious for the subject to log such events, especially when there may be a large number of them.

US 2013/0174077 discloses a medical information display apparatus that comprises a display unit for displaying information and a gesture input unit for detecting a gesture operation performed on a display surface of the display unit. An obtaining unit obtains medical information of a subject corresponding to the gesture operation performed on the display surface while a subject appearance image is displayed. However, although the subject can retrieve medical information in this way, it is a difficult and time consuming process for a medical professional to provide this medical information to the apparatus and it is also a burden on the subject to access the medical information using a display surface. Furthermore, there is added difficulty for both the medical professional and the subject due to the fact that they need to be able to translate a particular body part that they directly see or feel into a graphical representation in order to use of the apparatus appropriately. This costs extra time and also places a limitation on accuracy.

### SUMMARY OF THE INVENTION

As noted above, the limitations with existing techniques is that it can be a difficult and time consuming process for a medical professional to provide medical information to an apparatus for future retrieval by a subject and the subject is burdened by having to access the medical information using a display surface, which can also be a difficult and time consuming process. In addition, the existing techniques suffer from accuracy limitations. It would thus be valuable to have an improvement aimed at addressing these limitations.

Therefore, according to a first aspect, there is provided a system for storing audio and/or visual content of a user. The system comprises one or more processors configured to, in response to a detection of a gesture and/or voice command of a user, identify a location on a body of a subject indicated by the gesture and/or voice command. One or more processors are configured to control at least one memory to store an indication of the identified location on the body of the subject with audio and/or visual content of the user captured for a time period after or during which the gesture and/or voice command of the user is detected.

In some embodiments, the system may comprise one or more detectors configured to detect the gesture and/or voice command of the user and/or to capture the audio and/or visual content of the user.

In some embodiments, the time period may have a predefined duration or the time period may end in response to a detection of a further gesture and/or voice command of the user.

In some embodiments, the gesture may comprise a pointing gesture, a tapping gesture, a sliding gesture and/or an eye gesture. In some embodiments, the gesture may comprise a gesture of a body part of the user or a gesture of a device held by the user.

In some embodiments, the one or more processors may be configured to identify the location on the body of the subject by being configured to process visual content of the user captured for the time period during which the gesture and/or voice command of the user is detected, using body recognition to identify the location on the body of the subject indicated by the gesture and/or voice command. In addition or alternatively, in some embodiments, the one or more processors may be configured to identify the location on the body of the subject by being configured to use body-coupled communication to identify the location on the body of the subject indicated by the gesture and/or voice command.

According to a second aspect, there is provided a method for storing audio and/or visual content of a user. The method comprises, in response to a detection of a gesture and/or voice command of a user, identifying a location on a body of a subject indicated by the gesture and/or voice command and controlling at least one memory to store an indication of the identified location on the body of the subject with audio and/or visual content of the user captured for a time period after or during which the gesture and/or voice command of the user is detected.

According to a third aspect, there is provided a system for rendering audio and/or visual content of a user. The system comprises one or more processors configured to, in response to a detection of a gesture and/or voice command of a subject, identify the subject and a location on a body of the subject indicated by the gesture and/or voice command. One or more processors are configured to acquire, from at least one a memory, audio and/or visual content of a user stored with an indication of the identified location on the body of the identified subject and control one or more user interfaces to render the acquired audio and/or visual content of the user.

In some embodiments, the system may comprise: one or more detectors configured to detect the gesture and/or voice command of the subject; and/or one or more user interfaces configured to render the acquired audio and/or visual content of the user.

In some embodiments, one or more processors may be configured to control one or more user interfaces to render a visual representation of the subject indicating one or more locations on the body of the subject for which an indication is stored in the at least one memory. Alternatively or in addition, in some embodiments, one or more processors may be configured to control one or more user interfaces to render feedback indicative of a proximity of the gesture to a location on the body of the subject for which an indication is stored in the at least one memory.

In some embodiments, one or more processors may be configured to control one or more user interfaces to render the acquired audio and/or visual content of the user with a visual representation of the body of the identified subject.

In some embodiments, one or more processors may be configured to identify the subject by being configured to process visual content of the subject captured for a time period during which the gesture and/or voice command of the subject is detected, using camera-based recognition to identify the subject. In addition or alternatively, in some embodiments, one or more processors may be configured to identify the subject by being configured to process audio content of the subject captured for the time period during which the gesture and/or voice command of the subject is detected, using voice recognition to identify the subject. In addition or alternatively, in some embodiments, one or more processors may be configured to identify the subject by being configured to use body-coupled communication to identify the subject.

In some embodiments, one or more processors may be configured to identify the location on the body of the subject by being configured to process visual content of the subject captured for a time period during which the gesture and/or voice command of the subject is detected, using body recognition to identify the location on the body of the subject indicated by the gesture and/or voice command. In addition or alternatively, in some embodiments, one or more processors may be configured to identify the location on the body of the subject by being configured to use body-coupled communication to identify the location on the body of the subject indicated by the gesture and/or voice command.

According to a fourth aspect, there is provided a method for rendering audio and/or visual content of a user. The method comprises, in response to a detection of a gesture and/or voice command of a subject, identifying the subject and a location on a body of the subject indicated by the gesture and/or voice command. The method comprises acquiring, from at least one memory, audio and/or visual content of a user stored with an indication of the identified location on the body of the identified subject and controlling one or more user interfaces to render the acquired audio and/or visual content of the user.

According to a fifth aspect, there is provided a computer program product comprising a computer readable medium. The computer readable medium has computer readable code embodied therein. The computer readable code is configured such that, on execution by a suitable computer or processor, the computer or processor is caused to perform the method described earlier.

According to the aspects and embodiments described above, the limitations of existing techniques are addressed. In particular, according to the above-described aspects and embodiments, the manner in which a user (e.g. a medical professional) provides information is simplified, since all the user is required to do is perform a gesture and/or voice command for the information that they provide to be stored with an indication of the relevant location on the body of the subject to which the information relates. Similarly, the manner in which a subject (e.g. a patient) retrieves the information is also simplified, since all the subject is required to do is perform a gesture and/or voice command for the information on a relevant location on their body to be acquired and rendered. As the user and the subject can provide their gesture and/or voice command in relation to the relevant location on the actual body of the subject, the need for an additional device for inputting and retrieving information is eliminated. Instead, audio and/or visual content is naturally associated with a corresponding location on the body of the subject. The burden on the user and the subject is thus reduced and they are able to complete their respective tasks of providing information and retrieving information more efficiently through a simple gesture and/or voice command in relation to the relevant location on the actual body of the subject.

Moreover, according to the aspects and embodiments described above, a location on the body of the subject indicated by a gesture and/or voice command is identified such that the audio and/or visual content of a user relates specifically to the body anatomy and the information is thus more relevant and accurate. This enables improved logging of the body location of symptoms, for example, and also facilitates a better understanding and easier exploration of audio and/or visual content. In this way, the above-described aspects and embodiments facilitate in the diagnosis by a medical professional. There are thus provided improved systems and methods for storing audio and/or visual content of a user and for rendering audio and/or visual content of a user.

These and other aspects will be apparent from and elucidated with reference to the embodiment(s) described hereinafter.

### BRIEF DESCRIPTION OF THE DRAWINGS

Exemplary embodiments will now be described, by way of example only, with reference to the following drawings, in which:
Fig. 1 is a schematic illustration of a system according to an embodiment;
Fig. 2 is a flow chart illustrating a method according to an embodiment;
Fig. 3 is a schematic illustration of a system according to an embodiment;
Fig. 4 is a flow chart illustrating a method according to an embodiment; and
Fig. 5(a) and (b) illustrate example situations in which a system can be used.

### DETAILED DESCRIPTION OF EMBODIMENTS

As noted above, there is provided herein an improved system and method for storing audio and/or visual content of a user and an improved system and method for rendering audio and/or visual content of a user. Herein, references to a "user" may refer to a medical professional, such as a doctor, a nurse, a care giver, etc. Also, herein, references to a "subject" may refer to a patient. The gesture and/or voice command of a user described herein is a gesture and/or voice command aimed at the actual body of the subject.

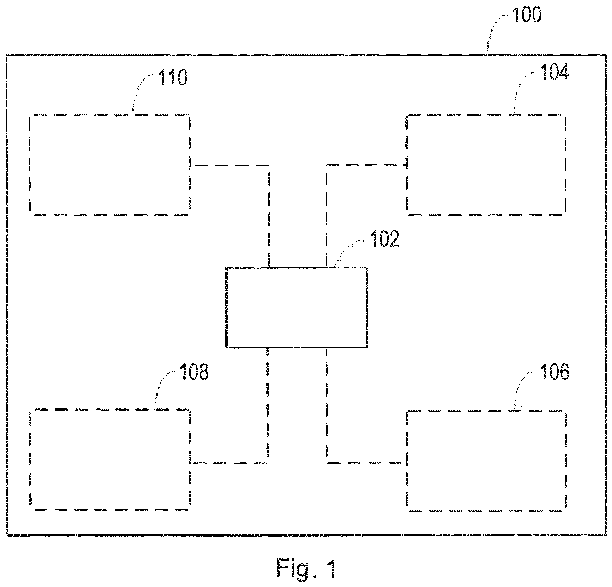

Fig. 1 illustrates a system 100 for storing audio and/or visual content of a user according to an embodiment. The system can be for use by a user, e.g. at a medical facility. A medical facility can be, for example, a general practitioner (GP) surgery, a hospital, a clinic, or any other medical facility. As illustrated in Fig. 1, the system 100 comprises one or more processors 102. The one or more processors 102 can be implemented in numerous ways, with software and/or hardware, to perform the various functions described herein.

In particular implementations, the one or more processors 102 can comprise a plurality of software and/or hardware modules, each configured to perform, or that are for performing, individual or multiple steps of the method described herein. The one or more processors 102 may comprise, for example, one or more microprocessors, one or more multicore processors and/or one or more digital signal processors (DSPs), one or more processing units, and/or one or more controllers (e.g. one or more microcontrollers) that may be configured or programmed (e.g. using software or computer program code) to perform the various functions described herein. The one or more processors 102 may be implemented as a combination of dedicated hardware (e.g. amplifiers, pre-amplifiers, analog-to-digital convertors (ADCs) and/or digital-to-analog convertors (DACs)) to perform some functions and one or more processors (e.g. one or more programmed microprocessors, DSPs and associated circuitry) to perform other functions.

Briefly, one or more processors 102 are configured to, in response to a detection of a gesture and/or voice command of a user, identify a location on (e.g. an area on or a part of) a body of a subject indicated by the gesture and/or voice command. Also, one or more processors 102 are configured to control at least one memory 104 to store an indication of the identified location on the body of the subject with audio and/or visual content of the user captured for a time period after or during which the gesture and/or voice command of the user is detected.

As illustrated in Fig. 1, in some embodiments, the system 100 may comprise at least one memory 104. Alternatively or in addition, in some embodiments, at least one memory 104 may be external to (e.g. separate to or remote from) the system 100. For example, another system may comprise at least one memory 104 according to some embodiments. In some embodiments, a hospital database may comprise at least one memory 104, at least one memory 104 may be a cloud computing resource, or similar. One or more processors 102 of the system 100 may be configured to communicate with and/or connect to at least one memory 104. The at least one memory 104 may comprise any type of non-transitory machine-readable medium, such as cache or system memory including volatile and non-volatile computer memory such as random access memory (RAM), static RAM (SRAM), dynamic RAM (DRAM), read-only memory (ROM), programmable ROM (PROM), erasable PROM (EPROM), and electrically erasable PROM (EEPROM). In some embodiments, at least one memory 104 can be configured to store program code that can be executed by one or more processors 102 of the system 100 to cause the system 100 to operate in the manner described herein.

Alternatively or in addition, at least one memory 104 can be configured to store information required by or resulting from the method described herein. For example, at least one memory 104 is configured to store the indication of the identified location on the body of the subject with audio and/or visual content of the user captured for the time period after or during which the gesture and/or voice command of the user is detected. At least one memory 104 may also be configured to store any other information, or any combination of information, required by or resulting from the method described herein. One or more processors 102 of the system 100 can be configured to control at least one memory 104 to store information required by or resulting from the method described herein.

As illustrated in Fig. 1, in some embodiments, the system 100 may comprise one or more detectors 106. Alternatively or in addition, in some embodiments, at least one detector 106 may be external to (e.g. separate to or remote from) the system 100. For example, another system may comprise at least one detector 106 according to some embodiments. One or more detectors 106 may be configured to detect the gesture and/or voice command of the user. Alternatively or in addition, one or more detectors 106 may be configured to capture the audio and/or visual content of the user. In some embodiments, at least one of the one or more detectors 106 configured to detect the gesture and/or voice command of the user may also be configured to capture the audio and/or visual content of the user. In other embodiments, the one or more detectors 106 configured to detect the gesture and/or voice command of the user and the one or more detectors 106 to capture the audio and/or visual content of the user may be different.

As illustrated in Fig. 1, in some embodiments, the system 100 may comprise at least one user interface 108. Alternatively or in addition, in some embodiments, at least one user interface 108 may be external to (e.g. separate to or remote from) the system 100. One or more processors 102 of the system 100 may be configured to communicate with and/or connect to at least one user interface 108. In some embodiments, one or more processors 102 of the system 100 can be configured to control at least one user interface 108 to operate in the manner described herein.

A user interface 108 can be configured to render (or output, display, or provide) information required by or resulting from the method described herein. For example, in some embodiments, one or more user interfaces 108 may be configured to render (or output, display, or provide) any one or more of the indication of the identified location on the body of the subject, the audio and/or visual content of the user captured for the time period after or during which the gesture and/or voice command of the user is detected, or any other information, or any combination of information, required by or resulting from the method described herein. Alternatively or in addition, one or more user interfaces 108 can be configured to receive a user input. For example, one or more user interfaces 108 may allow a user (e.g. the subject or another user) to manually enter information or instructions, interact with and/or control the system 100. Thus, one or more user interfaces 108 may be any one or more user interfaces that enable the rendering (or outputting, displaying, or providing) of information and/or enables a user to provide a user input.

For example, one or more user interfaces 108 may comprise one or more switches, one or more buttons, a keypad, a keyboard, a mouse, a display or display screen, a graphical user interface (GUI) such as a touch screen, an application (e.g. on a smart device such as a tablet, a smart phone, or any other smart device), or any other visual component, one or more speakers, one or more microphones or any other audio component, one or more lights (e.g. one or more light emitting diodes, LEDs), a component for providing tactile or haptic feedback (e.g. a vibration function, or any other tactile feedback component), an augmented reality device (e.g. augmented reality glasses, or any other augmented reality device), a smart device (e.g. a smart mirror, a tablet, a smart phone, a smart watch, or any other smart device), or any other user interface, or combination of user interfaces. In some embodiments, one or more user interfaces that are controlled to render information may be the same as one or more user interfaces that enable the user to provide a user input.

As illustrated in Fig. 1, in some embodiments, the system 100 may comprise at least one communications interface (or communications circuitry) 110. Alternatively or in addition, in some embodiments, at least one communications interface 110 may be external to (e.g. separate to or remote from) the system 100. A communications interface 110 can be for enabling the system 100, or components of the system 100 (e.g. one or more processors 102, one or more memories 104, one or more detectors 106, one or more user interfaces 108 and/or any other components of the system 100), to communicate with and/or connect to each other and/or one or more other components. For example, one or more communications interfaces 110 can be for enabling one or more processors 102 of the system 100 to communicate with and/or connect to one or more memories 104, one or more detectors 106, one or more user interfaces 108 and/or any other components of the system 100.

A communications interface 110 may enable the system 100, or components of the system 100, to communicate and/or connect in any suitable way. For example, one or more communications interfaces 110 may enable the system 100, or components of the system 100, to communicate and/or connect wirelessly, via a wired connection, or via any other communication (or data transfer) mechanism. In some wireless embodiments, for example, one or more communications interfaces 110 may enable the system 100, or components of the system 100, to use radio frequency (RF), Bluetooth, or any other wireless communication technology to communicate and/or connect.

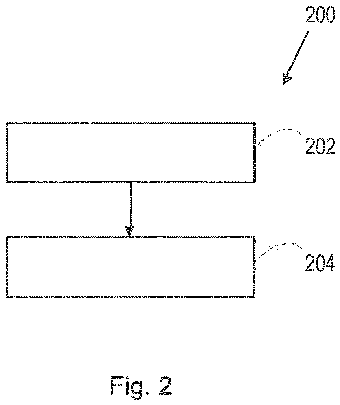

Fig. 2 illustrates a method 200 for storing audio and/or visual content of a user according to an embodiment. More specifically, Fig. 2 illustrates a method 200 of operating the system 100 described earlier with reference to Fig. 1 for storing audio and/or visual content of a user. The method 200 illustrated in Fig. 2 is a computer-implemented method. As described earlier, the system 100 described earlier with reference to Fig. 1 comprises one or more processors 102. The method 200 illustrated in Fig. 2 can generally be performed by or under the control of one or more processors 102 of the system 100 described earlier with reference to Fig. 1.

With reference to Fig. 2, at block 202, in response to a detection of a gesture and/or voice command of a user, a location on (e.g. an area on or a part of) a body of a subject indicated by the gesture and/or voice command is identified. More specifically, in response to a detection of a gesture and/or voice command of a user, one or more processors 102 of the system 100 identify the location on the body of the subject indicated by the gesture and/or voice command. The gesture and/or voice command of a user may be detected during a consultation with the user.

Herein, the gesture of the user may comprise a pointing gesture, a tapping gesture, a sliding gesture, an eye gesture (e.g. a gaze), or any other gesture, or any combination of gestures. In some embodiments, the gesture of the user may comprise a gesture of a body part (e.g. a finger, a hand, an eye, or any other body part) of the user or a gesture of a device (e.g. a pointing device or any other device) held by the user. The gesture of the user may include an interaction with (e.g. touching) the location on the body of the subject or the gesture of the user may be remote to the location of the body of the subject. Where the gesture of the user is remote to the location of the body of the subject, the gesture of the user may be within a predefined area around the location on the body of the subject (e.g. within 20 cm of the location on the body of the subject) to indicate that location on the body of the subject. Herein, the voice command of a user may comprise a spoken instruction of the user, such as "start recording" or "bookmark location".

One or more processors 102 of the system 100 may be configured to identify the location on the body of the subject indicated by the gesture and/or voice command of the user in any suitable way. In some embodiments, this may involve the use of a body localization system.

In some embodiments, one or more processors 102 of the system 100 may be configured to analyze visual content of the user to identify the location on the body of the subject indicated by a gesture of the user. For example, in some embodiments, the one or more processors 102 may be configured to identify the location on the body of the subject by being configured to process visual content of the user captured for the time period during which the gesture and/or voice command of the user is detected, using (e.g. three-dimensional) body recognition to identify the location on the body of the subject indicated by the gesture and/or voice command of the user. A person skilled in the art will be aware of suitable (e.g. three-dimensional) body recognition techniques and the manner in which they can be used to identify a location on (e.g. an area on or a part of) a body of a subject.

However, briefly, for example, a camera (e.g. a depth camera) may acquire an image of the body of the subject and the image may be processed by one or more processors 102 of the system 100 to identify body makers (e.g. distinguishable characteristics or features) on the body of the subject. These body markers in an image of the body of the subject can be used to reconstruct the skin surface of the body. A point on the skin can then be interpolated from the nearest body markers. The location on the body of the subject indicated by the gesture of the user can be identified by extrapolating the location of the gesture relative to the point on the skin. In some embodiments, an anatomical (e.g. medical) map extrapolated to the morphology of the subject may be overlaid on a recognized body skeleton to identify the location on the body of the subject indicated by the gesture of the user. In some embodiments, a camera (e.g. a depth camera) may be used to identify the gesture of the user.

The visual content of the user may be captured directly. For example, the visual content of the user may be acquired from a camera, such as a camera in the environment of the user (e.g. a wall-mounted camera) or on a device held by the user (e.g. a phone or computer). Alternatively or in addition, the visual content of the user may be captured indirectly. For example, the visual content of the user may be acquired from a mirror. The indirect capture of visual content of the user (e.g. via a mirror) can ensure that gestures of the user indicating a location on a body of a subject that are not otherwise visible (e.g. a location on the back of a body of a subject) can still be detected. One or more processors 102 of the system 100 may process the visual content of the user captured indirectly using body mapping.

Alternatively or in addition, in some embodiments, one or more processors 102 of the system 100 may be configured to identify the location on the body of the subject by being configured to use body-coupled communication to identify the location on the body of the subject indicated by the gesture and/or voice command. A person skilled in the art will be aware of suitable body-coupled communication techniques and the manner in which they can be used to identify a location on (e.g. an area on or a part of) a body of a subject.

However, briefly, in body-coupled communication, the body of the user and the body of the subject serve as communication channels. An electrical signal is induced into the body of the user (e.g. via a wearable sensor, such as a wrist-worn sensor) and is transmitted through the body of the user and into the body of the subject when the user touches the subject (e.g. with their finger). The resulting (e.g. galvanic or capacitive) coupling is measured by one or more sensors worn by the subject. The measured coupling will be different depending on the location on the body (e.g. the body part) of the subject that the user touches. A body map of the coupling can be (e.g. partially) calculated. The body map may be (e.g. partially) pre-calibrated. The body map may map coupling values to locations on the body of the subject. The one or more processors 102 of the system 100 can identify the location on the body (e.g. the body part) of the subject that is touched by the user at a given moment by comparing an acquired coupling value to the mapped coupling values.

Returning back to Fig. 2, at block 204, at least one memory 104 is controlled to store an indication of the identified location on the body of the subject with audio and/or visual content of the user captured for a time period after or during which the gesture and/or voice command of the user is detected. More specifically, one or more processors 102 of the system 100 control at least one memory 104 in this way. Thus, the identified location on the body of the subject can be bookmarked and stored with audio and/or visual content of the user associated with the identified location on the body of the subject. In some examples, the location on the body of the subject indicated by the gesture and/or voice command of the user may be on the left part of the chest of the user for a heart disease, on the head of the user for a headache, on the arm for a blood pressure measurement, and so on. There may be an indication of one or more locations on the body of the subject stored with audio and/or visual content of the user, e.g. a number in a range from 1 to 10.

In some embodiments, one or more processors 102 can be configured to automatically control at least one memory 104 to store the indication of the identified location on the body of the subject with the audio and/or visual content of the user in response to the (e.g. upon) detection of the gesture and/or voice command of the subject. In some embodiments, the indication of the identified location on the body of the subject may be stored with audio and/or visual content of the user captured from different times (e.g. at each consultation or each day), such that the user can view the audio and/or visual content to observe changes over time. The at least one memory 104 may store an indication of a plurality of identified locations on the body of the subject with associated audio and/or visual content of the user. In some embodiments, the indication of the identified location on the body of the subject may be stored with its associated (or respective) audio and/or visual content of the user in the at least one memory 104 in the form of a standard directory, such as a two-dimensional representation, a list (e.g. an indexed list), a look-up table, etc.

In some embodiments, one or more processors 102 of the system 100 can be configured to, while the audio and/or visual content of the user is captured, control at least one user interface 108 to render other relevant information (e.g. medical information, such as scan images, anatomical representations, etc.). The at least one user interface 108 may, for example, comprise a screen or a virtual space. In this way, useful information can be provided to the subject to supplement the audio and/or visual content of the user as that audio and/or visual content is captured.

In some embodiments, the time period after or during which the gesture and/or voice command of the user is detected may have a predefined duration. In some embodiments, the time period after which the gesture and/or voice command of the user is detected may begin in response to the detection of the gesture and/or voice command of the user. In some embodiments, the time period after which the gesture and/or voice command of the user is detected may end after a predefined amount of time or after a predefined amount of audio and/or visual content (e.g. a predefined amount of images and/or sentences) is captured. In some embodiments, the time period after which the gesture and/or voice command of the user is detected may end after a detection of a further gesture and/or voice command of the user. The further gesture may, for example, comprise the user ceasing to perform the original gesture (e.g. no longer touching or pointing at the identified location on the body of the subject) or a visual instruction to stop capturing audio and/or visual content (e.g. a tapping action of the user, or an extra tapping action of the user when the original gesture is a tapping action of the user, directed toward the identified location on the body of the subject). The further voice command may, for example, comprise the user ceasing to perform the original voice command (e.g. no longer speaking) or a spoken instruction to stop capturing audio and/or visual content, such as "stop recording". In some embodiments, the audio and/or visual content may be captured continuously and the time period during which the gesture and/or voice command of the user is detected may be isolated, e.g. using audio and/or visual analysis (such as gesture recognition and/or voice recognition).

The audio content of the user referred to herein may, for example, comprise the voice of the user. The visual content of the user referred to herein may, for example, comprise one or more images and/or one or more videos of the user. The audio and/or visual content of the user referred to herein can comprise (e.g. personal) medical information or health data. The medical information or health data can, for example, include explanations from a medical professional, e.g. regarding the location of a disease, one or more symptoms, a recommended treatment, the location of one or more monitoring points, an indication of where to position a blood pressure cuff, etc.

Although not illustrated in Fig. 2, in some embodiments, one or more processors 102 of the system 100 may be configured to identify the user. The user may be identified in any suitable way.

For example, in some embodiments, one or more processors 102 of the system 100 may be configured to identify the user by being configured to process visual content of the user captured for a time period during which the gesture and/or voice command of the user is detected, using camera-based recognition to identify the user. A person skilled in the art will be aware of suitable three-dimensional skeletal recognition camera-based recognition techniques and the manner in which they can be used to identify a user. However, briefly, for example, a facial recognition technique can be used to identify the user. This may comprise one or more processors 102 of the system 100 comparing one or more images obtained by a camera (e.g. in the environment of the user or of a device held by the user) with images of faces in a memory 104, each stored with an indication of the identity of a user to which the face in the image belongs. The comparison may comprise identifying one or more facial features in the one or more obtained images and determining whether they match corresponding facial features in the images of faces stored in the memory 104. Where there is a match, the user is identified by way of the identity that is stored with the image that comprises the matching facial features.

Alternatively or in addition, in some embodiments, one or more processors 102 of the system 100 may be configured to identify the user by being configured to process audio content of the user captured for a time period during which the gesture and/or voice command of the user is detected, using voice (or speech) recognition to identify the user. A person skilled in the art will be aware of suitable voice (or speech) recognition techniques and the manner in which they can be used to identify a user. However, briefly, for example, one or more processors 102 of the system 100 may use pattern recognition to process a captured voice print and compare the captured voice print to reference voice prints, each stored in a memory 104 with an indication of a user to which the reference voice print corresponds, to identify a user.

Alternatively or in addition, in some embodiments, one or more processors 102 of the system 100 may be configured to identify the user by being configured to use body-coupled communication to identify the user. A person skilled in the art will be aware of suitable body-coupled communication techniques and the manner in which they can be used to identify a user. However, briefly, for example, the user may wear a device (e.g. a tag), which is configured to induce an electric signal into their body that can be identified. The device configured to induce the electric signal acts as a personal identifier for the user. The electrical signal may be detected and processed to identify the user.

Alternatively or in addition, in embodiments where the gesture comprises a gesture of a device (e.g. a pointing device) held by the user, the device may be used to identify the user. In some embodiments where the user is identified, one or more processors 102 of the system 100 may be configured to control at least one memory 104 to store an indication of the identity of the user with the audio and/or visual content of the user.

Although also not illustrated in Fig. 2, in some embodiments, the processor 102 of the system 100 may be configured to control at least one memory 104 to store additional information associated with one or more locations (which may include the identified location) on the body of the subject. The additional information may be (e.g. automatically) retrieved from a (e.g. generic) database and/or received via at least one user interface 108 (e.g. a GUI). The database may, for example, comprise an electronic health record (EHR) or an electronic medical record (EMR) from which the additional information may be retrieved. In some embodiments, the user may input the additional information via at least one user interface 108. In some embodiments, at least one user interface 108 may be configured to render a visual representation of the body of the identified subject on which the user can tag (or select) one or more locations on the body of the subject to input the additional information associated with those one or more locations. The additional information is defined as information in addition to the audio and/or visual content of the user. The additional information can thus supplement the audio and/or visual content of the user. The additional information can be (e.g. automatically) linked to the relevant location on the body of the subject. In this way, a directory of information can be associated with one or more locations (which may include the identified location) on the body of the subject.

In some embodiments, the user may input additional information while or after performing the gesture and/or voice command, such that the processor 102 of the system 100 associates the additional information to the identified location on the body of the subject indicated by the gesture and/or voice command. In an example where the user is performing a gesture with one hand, the user may input this additional information via a user interface 108 using their other hand or with a voice command. Alternatively or in addition, in some embodiments, the additional information may be provided subsequent to (e.g. at a later time to) the capture of the audio and/or visual content of the user. Examples of additional information include, but are not limited to, medical information on the subject (or health data on the subject), which may be in the form of one or more explanations from the user, one or more medical images, one or more medical videos, one or more medical (e.g. diagnosis) reports, one or more anatomical visualization (or representation), a description of one or more treatment options, one or more prescriptions, a link to one or more support groups, one or more questions for the subject (e.g. regarding their next consultation, feelings, symptoms, etc.), or any other additional information, or any combination of additional information.

In an example, additional information (such as an explanatory anatomical representation) may be queried from a medical database by a medical professional, rendered on a display and explained during a consultation with a subject. The additional information is associated to the relevant location on the body of the subject, e.g. by a gesture of the medical professional. In another example, a movement that a subject has to perform for rehabilitation may be shown to the subject by a medical professional, visually captured by a camera and associated to the relevant location on the body of the subject. In this example, the visually captured movement is the additional information.

In some embodiments, any one or more of the identified location on the body of the subject indicated by the gesture and/or voice command of the user and the associated audio and/or visual content of the user may be (e.g. automatically) updated over time, e.g. as a medical condition (e.g. disease) migrates. The identified location on the body of the subject indicated by the gesture and/or voice command may be updated by the processor 102 of the system 100 analyzing audio and/or visual content of the user to detect when the gesture and/or voice command of the user indicates a shift in the identified location on the body of the subject. The processor 102 of the system 100 can be configured to control at least one memory 104 to store the updated identified location on the body of the subject. The processor 102 of the system 100 can be configured to control at least one memory 104 store the updated identified location on the body of the subject with any one or more of the audio and/or visual content of the user captured for the time period after or during which the original gesture and/or voice command of the user is detected and the audio and/or visual content of the user captured for a time period after or during which the gesture and/or voice command of the user indicating a shift in the identified location on the body of the subject is detected.

In this way, it is possible to observe the evolution of the identified location and associated audio and/or visual data. Thus, for example, it may be possible to observe a medical condition (e.g. a disease) improving or worsening. As such, by updating any one or more of the identified location on the body of the subject and the associated audio and/or visual content of the user, the subsequent engagement of the subject may be stimulated.

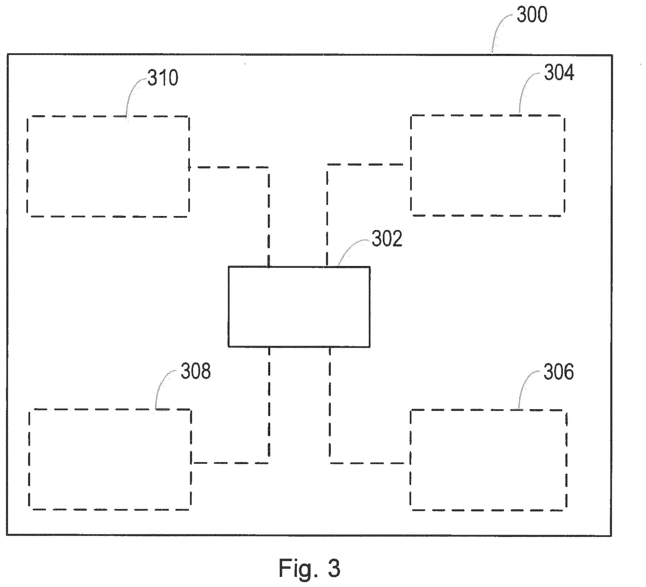

Fig. 3 illustrates a system 300 for rendering audio and/or visual content of a user according to an embodiment. The system can be for use by a subject, e.g. in their own environment, such as at home. As illustrated in Fig. 3, the system 300 comprises one or more processors 302. The one or more processors 302 can be implemented in numerous ways, with software and/or hardware, to perform the various functions described herein.

In particular implementations, the one or more processors 302 can comprise a plurality of software and/or hardware modules, each configured to perform, or that are for performing, individual or multiple steps of the method described herein. The one or more processors 302 may comprise, for example, one or more microprocessors, one or more multicore processors and/or one or more digital signal processors (DSPs), one or more processing units, and/or one or more controllers (e.g. one or more microcontrollers) that may be configured or programmed (e.g. using software or computer program code) to perform the various functions described herein. The one or more processors 302 may be implemented as a combination of dedicated hardware (e.g. amplifiers, pre-amplifiers, analog-to-digital convertors (ADCs) and/or digital-to-analog convertors (DACs)) to perform some functions and one or more processors (e.g. one or more programmed microprocessors, DSPs and associated circuitry) to perform other functions.

Briefly, one or more processors 302 are configured to, in response to a detection of a gesture and/or voice command of a subject, identify the subject and a location on (e.g. an area on or a part of) a body of the subject indicated by the gesture and/or voice command. One or more processors 302 are also configured to acquire, from at least one a memory 304, audio and/or visual content of a user stored with an indication of the identified location on the body of the identified subject and control one or more user interfaces 308 to render the acquired audio and/or visual content of the user. As mentioned earlier, herein, references to a "user" may refer to a medical professional, such as a doctor, a nurse, a care giver, etc. Also, herein, references to a "subject" may refer to a patient.

As illustrated in Fig. 3, in some embodiments, the system 300 may comprise at least one memory 304. Alternatively or in addition, in some embodiments, at least one memory 304 may be external to (e.g. separate to or remote from) the system 300. For example, another system may comprise at least one memory 304 according to some embodiments. In some embodiments, a hospital database may comprise at least one memory 304, at least one memory 304 may be a cloud computing resource, or similar. In some embodiments, the at least one memory 304 of the system 300 for rendering audio and/or visual content of a user may be the same as the at least one memory 104 of the system 100 for storing audio and/or visual content of a user. For example, the at least one memory 104, 304 may be a central memory according to some embodiments. In other embodiments, the at least one memory 304 of the system 300 for rendering audio and/or visual content of a user and the at least one memory 104 of the system 100 for storing audio and/or visual content of a user may be different.

One or more processors 302 of the system 300 may be configured to communicate with and/or connect to at least one memory 304. The at least one memory 304 may comprise any type of non-transitory machine-readable medium, such as cache or system memory including volatile and non-volatile computer memory such as random access memory (RAM), static RAM (SRAM), dynamic RAM (DRAM), read-only memory (ROM), programmable ROM (PROM), erasable PROM (EPROM), and electrically erasable PROM (EEPROM). In some embodiments, at least one memory 304 can be configured to store program code that can be executed by one or more processors 302 of the system 300 to cause the system 300 to operate in the manner described herein.

Alternatively or in addition, at least one memory 304 can be configured to store information required by or resulting from the method described herein. For example, at least one memory 304 is configured to store audio and/or visual content of a user with an indication of a location on (e.g. an area on or a part of) a body of a subject. At least one memory 304 may also be configured to store any other information, or any combination of information, required by or resulting from the method described herein. One or more processors 302 of the system 300 can be configured to control at least one memory 304 to store information required by or resulting from the method described herein.

As illustrated in Fig. 3, in some embodiments, the system 300 may comprise one or more detectors 306. Alternatively or in addition, in some embodiments, at least one detector 306 may be external to (e.g. separate to or remote from) the system 300. For example, another system may comprise at least one detector 306 according to some embodiments. One or more detectors 306 may be configured to detect the gesture and/or voice command of the subject.

As illustrated in Fig. 3, in some embodiments, the system 300 may comprise at least one user interface 308. Alternatively or in addition, in some embodiments, at least one user interface 308 may be external to (e.g. separate to or remote from) the system 300. For example, another system may comprise at least one user interface 308 according to some embodiments. One or more processors 302 of the system 300 may be configured to communicate with and/or connect to at least one user interface 308. In some embodiments, one or more processors 302 of the system 300 can be configured to control at least one user interface 308 to operate in the manner described herein.

A user interface 308 can be configured to render (or output, display, or provide) information required by or resulting from the method described herein. For example, in some embodiments, one or more user interfaces 308 may be configured to render (or output, display, or provide) any one or more of an indication of the identified subject, an indication of the identified location on the body of the subject, the acquired audio and/or visual content of the user, or any other information, or any combination of information, required by or resulting from the method described herein. Alternatively or in addition, one or more user interfaces 308 can be configured to receive a user input. For example, one or more user interfaces 308 may allow a user (e.g. the subject or another user) to manually enter information or instructions, interact with and/or control the system 300. Thus, one or more user interfaces 308 may be any one or more user interfaces that enable the rendering (or outputting, displaying, or providing) of information and/or that enable a user to provide a user input.

For example, one or more user interfaces 308 may comprise one or more switches, one or more buttons, a keypad, a keyboard, a mouse, a display or display screen, a graphical user interface (GUI) such as a touch screen, an application (e.g. on a smart device such as a tablet, a smart phone, or any other smart device), or any other visual component, one or more speakers, one or more microphones or any other audio component, one or more lights (e.g. one or more light emitting diodes, LEDs), a component for providing tactile or haptic feedback (e.g. a vibration function, or any other tactile feedback component), an augmented reality device (e.g. augmented reality glasses, or any other augmented reality device), a smart device (e.g. a smart mirror, a tablet, a smart phone, a smart watch, or any other smart device), or any other user interface, or combination of user interfaces. In some embodiments, one or more user interfaces that are controlled to render information may be the same as one or more user interfaces that enable the user to provide a user input.

As illustrated in Fig. 3, in some embodiments, the system 300 may comprise at least one communications interface (or communications circuitry) 310. Alternatively or in addition, in some embodiments, at least one communications interface 310 may be external to (e.g. separate to or remote from) the system 300. A communications interface 310 can be for enabling the system 300, or components of the system 300 (e.g. one or more processors 302, one or more memories 304, one or more detectors 106, one or more user interfaces 308 and/or any other components of the system 300), to communicate with and/or connect to each other and/or one or more other components. For example, one or more communications interfaces 310 can be for enabling one or more processors 302 of the system 300 to communicate with and/or connect to one or more memories 304, one or more detectors 106, one or more user interfaces 308 and/or any other components of the system 300.

A communications interface 310 may enable the system 300, or components of the system 300, to communicate and/or connect in any suitable way. For example, one or more communications interfaces 310 may enable the system 300, or components of the system 300, to communicate and/or connect wirelessly, via a wired connection, or via any other communication (or data transfer) mechanism. In some wireless embodiments, for example, one or more communications interfaces 310 may enable the system 300, or components of the system 300, to use radio frequency (RF), Bluetooth, or any other wireless communication technology to communicate and/or connect.

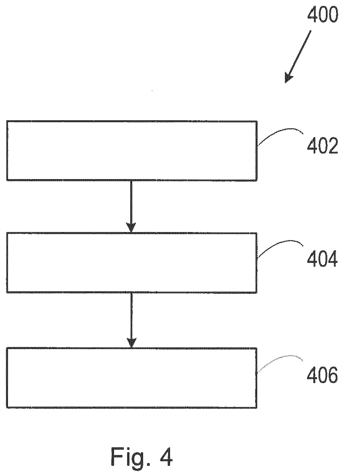

Fig. 4 illustrates a method 400 for rendering audio and/or visual content of a user according to an embodiment. More specifically, Fig. 4 illustrates a method 400 of operating the system 300 described earlier with reference to Fig. 3 for rendering audio and/or visual content of a user. The method 400 illustrated in Fig. 4 is a computer-implemented method. As described earlier, the system 300 described earlier with reference to Fig. 3 comprises one or more processors 302. The method 400 illustrated in Fig. 4 can generally be performed by or under the control of one or more processors 302 of the system 300 described earlier with reference to Fig. 3.

With reference to Fig. 4, at block 402, in response to a detection of a gesture and/or voice command of a subject, the subject and a location on (e.g. an area on or a part of) a body of the subject indicated by the gesture and/or voice command is identified. More specifically, one or more processors 302 of the system 300, in response to a detection of a gesture and/or voice command of a subject, identify the subject and the location on the body of the subject indicated by the gesture and/or voice command.

Herein, the gesture of the subject may comprise a pointing gesture, a tapping gesture, a sliding gesture, an eye gesture (e.g. a gaze), or any other gesture, or any combination of gestures. In some embodiments, the gesture of the subject may comprise a gesture of a body part (e.g. a finger, a hand, an eye, or any other body part) of the subject or a gesture of a device (e.g. a pointing device or any other device) held by the subject. The gesture of the subject may include an interaction with (e.g. touching) the location on the body of the subject or the gesture may be remote to the location of the body of the subject. Where the gesture of the subject is remote to the location of the body of the subject, the gesture of the subject may be within a predefined area around the location on the body of the subject (e.g. within 20 cm of the location on the body of the subject) to indicate that location on the body of the subject. Herein, the voice command of a subject may comprise a spoken instruction of the subject, such as "start playback".

The one or more processors 302 of the system 300 may be configured to identify the subject in any suitable way.

For example, in some embodiments, one or more processors 302 of the system 300 may be configured to identify the subject by being configured to process visual content of the subject captured for a time period during which the gesture and/or voice command of the subject is detected, using camera-based recognition to identify the subject. A person skilled in the art will be aware of suitable camera-based recognition techniques and the manner in which they can be used to identify a subject. However, briefly, for example, a facial recognition technique can be used to identify the subject. This may comprise one or more processors 102 of the system 100 comparing one or more images obtained by a camera (e.g. in the environment of the subject or of a device held by the subject) with images of faces in a memory 104, each stored with an identity of a subject to which the face in the image belongs. The comparison may comprise identifying one or more facial features in the one or more obtained images and determining whether they match corresponding facial features in the images of faces stored in the memory 104. Where there is a match, the subject is identified by way of the identity that is stored with the image that comprises the matching facial features.

Alternatively or in addition, in some embodiments, one or more processors 302 of the system may be configured to identify the subject by being configured to process audio content of the subject captured for a time period during which the gesture and/or voice command of the subject is detected, using voice (or speech) recognition to identify the subject. A person skilled in the art will be aware of suitable voice (or speech) recognition techniques and the manner in which they can be used to identify a subject. However, briefly, for example, one or more processors 102 of the system 100 may use pattern recognition to process a captured voice print and compare the captured voice print to reference voice prints, each stored in a memory 104 with an indication of a subject to which the reference voice print corresponds, to identify a subject.

Alternatively or in addition, in some embodiments, one or more processors 302 of the system 300 may be configured to identify the subject by being configured to use body-coupled communication to identify the subject. A person skilled in the art will be aware of suitable body-coupled communication techniques and the manner in which they can be used to identify a subject. However, briefly, for example, the subject may wear a device (e.g. a tag), which is configured to induce an electric signal into their body that can be identified. The device configured to induce the electric signal acts as a personal identifier for the subject. The electrical signal may be detected and processed to identify the subject.

Alternatively or in addition, in embodiments where the gesture comprises a gesture of a device (e.g. a pointing device) held by the subject, the device may be used to identify the subject.

One or more processors 302 of the system 300 may be configured to identify the location on the body of the subject indicated by the gesture and/or voice command of the subject in any suitable way. In some embodiments, this may involve the use of a body localization system.

In some embodiments, one or more processors 302 of the system 300 may be configured to analyze visual content of the subject to identify the location on the body of the subject indicated by a gesture of the subject. For example, in some embodiments, the one or more processors 302 may be configured to identify the location on the body of the subject by being configured to process visual content of the subject captured for a time period during which the gesture and/or voice command of the subject is detected, using (e.g. three-dimensional) body recognition to identify the location on the body of the subject. A person skilled in the art will be aware of suitable (e.g. three-dimensional) body recognition techniques and the manner in which they can be used to identify a location on (e.g. an area on or a part of) a body of a subject.

However, briefly, for example, a camera (e.g. a depth camera) may acquire an image of the body of the subject and the image may be processed by one or more processors 102 of the system 100 to identify body makers (e.g. distinguishable characteristics or features) on the body of the subject. These body markers in an image of the body of the subject can be used to reconstruct the skin surface of the body. A point on the skin can then be interpolated from the nearest body markers. The location on the body of the subject indicated by the gesture of the subject can be identified by extrapolating the location of the gesture relative to the point on the skin. In some embodiments, an anatomical (e.g. medical) map extrapolated to the morphology of the subject may be overlaid on a recognized body skeleton to identify the location on the body of the subject indicated by the gesture of the subject. In some embodiments, a camera (e.g. a depth camera) may be used to identify the gesture of the subject.

The visual content of the subject may be captured directly. For example, the visual content of the subject may be acquired from a camera, such as a camera in the environment of the subject (e.g. a wall-mounted camera) or on a device held by the subject (e.g. a phone or computer). Alternatively or in addition, the visual content of the subject may be captured indirectly. For example, the visual content of the subject may be acquired from a mirror. The indirect capture of visual content of the subject (e.g. via a mirror) can ensure that gestures of the subject indicating a location on a body of a subject that is not otherwise visible (e.g. a location on the back of a body of a subject) can still be detected. One or more processors 102 of the system 100 may process the visual content of the subject captured indirectly using body mapping.

Alternatively or in addition, in some embodiments, one or more processors 302 of the system 300 may be configured to identify the location on the body of the subject by being configured to use body-coupled communication to identify the location on the body of the subject. A person skilled in the art will be aware of suitable body-coupled communication techniques and the manner in which they can be used to identify a location on (e.g. an area on or a part of) a body of a subject.

However, briefly, in body-coupled communication, the body of the subject serves as a communication channel. An electrical signal is induced into the body of the subject (e.g. via a wearable sensor, such as a wrist-worn sensor) and is transmitted through the body of the subject when the subject touches a location on their body (e.g. with their finger). The resulting (e.g. galvanic or capacitive) coupling is measured by one or more sensors worn on the body of the subject. The measured coupling will be different depending on the location on the body (e.g. the body part) of the subject that the subject touches. A body map of the coupling can be (e.g. partially) calculated. The body map may be (e.g. partially) pre-calibrated. The body map may map coupling values to locations on the body of the subject. The one or more processors 102 of the system 100 can identify the location on the body (e.g. the body part) of the subject that is touched by the subject at a given moment by comparing an acquired coupling value to the mapped coupling values.

Returning back to Fig. 4, at block 404, audio and/or visual content of a user stored with an indication of the identified location on the body of the identified subject is acquired from at least one memory 304. More specifically, one or more processors 302 of the system 300 acquire, from at least one memory 304, audio and/or visual content of a user stored with an indication of the identified location on the body of the identified subject.

At block 406 of Fig. 4, one or more user interfaces 308 are controlled to render the acquired audio and/or visual content of the user. More specifically, one or more processors 302 of the system 300 control one or more user interfaces 308 to render the acquired audio and/or visual content of the user. For example, one or more user interfaces 308 may comprise a speaker and/or a display via which to render the acquired audio and/or visual content of the user. Thus, audio and/or visual content of the user, which may comprise medical information or health data for the subject as described earlier, is associated with a location on the body of the subject and can be rendered in order to allow the subject to easily navigate their health data by activating (with the gesture and/or voice command) the relevant body part.

In some embodiments, one or more processors 302 can be configured to automatically render the acquired audio and/or visual content of the user in response to the (e.g. upon) detection of the gesture and/or voice command of the subject. In some embodiments, one or more processors 302 can be configured to control one or more user interfaces 308 to render the acquired audio and/or visual content of the user with a visual representation of the body of the identified subject. In some embodiments, the one or more processors 302 can be configured to control one or more user interfaces 308 to overlay acquired visual content of the user (and any additional information) over the visual representation of the body of the identified subject. In some embodiments, the one or more processors 302 can be configured to control one or more user interfaces 308 to overlay acquired visual content of the user (and any additional information) over the actual body of the identified subject (e.g. using augmented reality, AR). The acquired visual content of the user can be rendered on any user interface 308, e.g. on a display, on a screen, in a virtual or augmented reality space, or in any other way.

Although not illustrated in Fig. 4, in some embodiments, one or more processors 302 may be configured to control one or more user interfaces 308 (e.g. a display or augmented reality device) to render a visual representation of the subject indicating one or more locations on the body of the subject for which an indication is stored in the at least one memory 304. The rendered visual representation of the subject can effectively provides a map of the one or more locations on the body of the subject for which an indication is stored in the at least one memory 304. In some embodiments, one or more processors 302 may be configured to render a live position of the body part of the subject or the device held by the subject to perform the gesture with the visual representation of the subject indicating one or more locations on the body of the subject for which an indication is stored in the at least one memory 304. In this way, the subject can be provided with visual guidance (e.g. with reference to the body part or device that they use for the gesture) to revisit saved points on their body and retrieve associated audio and/or visual content. In a similar manner, one or more processors 302 may be configured to control one or more user interfaces 308 (e.g. a speaker) to render audio guidance to direct the subject to one or more locations on the body of the subject for which an indication is stored in the at least one memory 304.

Alternatively or in addition, in some embodiments, one or more processors 302 may be configured to control one or more user interfaces 308 to render feedback indicative of a proximity of the gesture of the subject to a location on (e.g. an area on or a part of) the body of the subject for which an indication is stored in the at least one memory 304. For example, one or more processors 302 may be configured to control one or more tactile or haptic feedback components to render tactile or haptic feedback (such as a vibration) indicative of a proximity of the gesture to a location on the body of the subject for which an indication is stored in the at least one memory 304. The tactile or haptic feedback may, for example, be rendered through a wearable (e.g. on the finger or the hand of the subject) or through a handheld device (e.g. a smart phone). Alternatively or in addition, for example, the one or more processors 302 may be configured to control one or more light sources to render luminous feedback (such as a luminous intensity or a color shift) indicative of a proximity of the gesture to a location on the body of the subject for which an indication is stored in the at least one memory 304. The feedback may be rendered as the gesture of the subject is within a predefined distance (e.g. 20cm) of a location the body of the subject for which an indication is stored in the at least one memory 304. In some embodiments, the feedback that is rendered may become more intense as the gesture of the subject comes closer to a location the body of the subject for which an indication is stored in the at least one memory 304. In this way, the subject can be provided with guidance to locate and activate bookmarks on their body and retrieve associated audio and/or visual content.

In some embodiments, the subject may input data (such as one or more symptoms) associated with one or more locations on the body of the subject for which an indication is stored in the at least one memory 304. The subject can input such data via at least one user interface 308. In some embodiments where the visual representation of the subject is rendered, the subject may input the data as annotations to the visual representation. The processor 302 can be configured to acquire input data via at least one user interface 308 and control at least one memory 304 to store the input data.

In some embodiments, the system 300 for rendering audio and/or visual content of a user may also be used for checking symptoms of the subject. For example, if a subject is suffering from a symptom (e.g. a headache), the subject can describe the symptoms by way of a gesture and/or voice command. For example, the subject may describe the sharpness or intensity of pain by a tapping gesture (e.g. the number of times the tapping gesture is performed may rate the pain), a direction of pain by a sliding gesture, or any other symptom by way of any other gesture. Alternatively or in addition, the subject may describe the sharpness or intensity of pain, the direction of pain, or any other symptom verbally. The processor 302 of the system 300 may be configured to process the indicated symptoms.

In some embodiments, the processor 302 of the system 300 may be configured to control at least one user interface 308 to render one or more questions to ask for confirmation or details of the symptoms of the subject. In this way, the urgency of a potential medical condition associated with the symptoms of the subject can be assessed. In some embodiments, the processor 302 of the system 300 may be configured to control at least one user interface 308 to render notifications relating to the symptoms, such as an indication of an event that may be related to the symptoms, an option to store the symptoms with an indication of the corresponding location on the body of the subject, or any other notification relating to the symptoms. In this way, the system 300 can be pro-active.

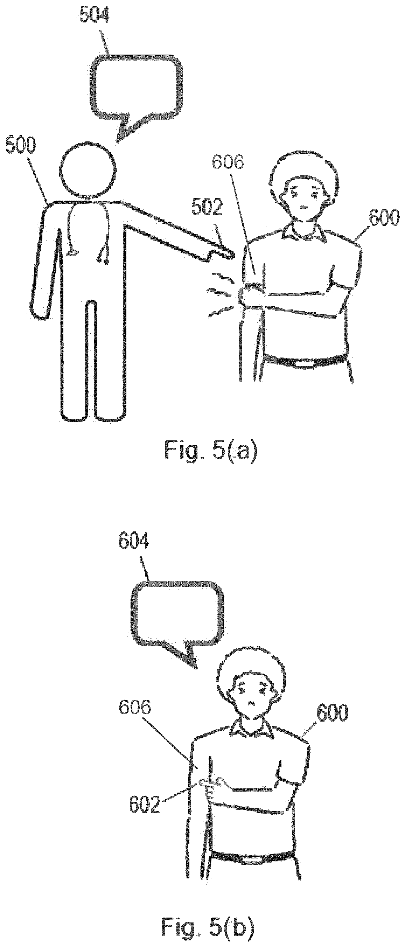

Fig. 5(a) illustrates a situation in which the system 100 for storing audio and/or visual content of a user may be used. In this example, the user 500 is a medical professional (e.g. a doctor) and the subject 600 is a patient. As illustrated in Fig. 5(a), the user 500 performs a gesture 502 and/or a voice command 504. In this example, the gesture is a pointing gesture. As described earlier, in response to a detection of the gesture 502 and/or voice command 504 of the user 500, one or more processors 102 of the system 100 (not illustrated in Fig. 5(a)) identify a location 606 on a body of a subject 600 indicated by the gesture 502 and/or voice command 504. In this example, the location 606 on the body of the subject 600 indicated by the gesture 502 and/or voice command 504 is on the arm of the subject 600. As also described earlier, one or more processors 102 of the system 100 control at least one memory 104 (not illustrated in Fig. 5(a)) to store an indication of the identified location 606 on the body of the subject 600 with audio and/or visual content of the user 500 captured for a time period after or during which the gesture 502 and/or voice command 504 of the user 500 is detected.

Fig. 5(b) illustrates a situation in which the system 300 for rendering audio and/or visual content of a user may be used. In this example, the subject 600 is a patient. As illustrated in Fig. 5(b), the subject 600 performs a gesture 602 and/or a voice command 604. In this example, the gesture is a pointing gesture. As described earlier, in response to a detection of a gesture 602 and/or voice command 604 of a subject 600, one or more processors 302 of the system 300 (not illustrated in Fig. 5(b)) identify the subject 600 and a location 606 on the body of the subject indicated by the gesture 602 and/or voice command 604. As also described earlier, one or more processors 302 of the system 300 acquire, from at least one a memory 304 (not illustrated in Fig. 5(b)), audio and/or visual content of a user 500 stored with an indication of the identified location 606 on the body of the identified subject 600 and control one or more user interfaces 308 (not illustrated in Fig. 5(b)) to render the acquired audio and/or visual content of the user 500. There is thus provided a multimodal system in which a location on a body of a subject can be bookmarked by way of a gesture and/or voice command of a user and through which a subject can subsequently activate replay of audio and/or visual content of the user, and possibly perform other specific actions (e.g. record associated symptoms).

There is also provided a computer program product comprising a computer readable medium. The computer readable medium has computer readable code embodied therein. The computer readable code is configured such that, on execution by a suitable computer or processor, the computer or processor is caused to perform the method described herein. The computer readable medium may be, for example, any entity or device capable of carrying the computer program product. For example, the computer readable medium may include a data storage, such as a ROM (such as a CD-ROM or a semiconductor ROM) or a magnetic recording medium (such as a hard disk). Furthermore, the computer readable medium may be a transmissible carrier, such as an electric or optical signal, which may be conveyed via electric or optical cable or by radio or other means. When the computer program product is embodied in such a signal, the computer readable medium may be constituted by such a cable or other device or means. Alternatively, the computer readable medium may be an integrated circuit in which the computer program product is embedded, the integrated circuit being adapted to perform, or used in the performance of, the method described herein.

There is thus provided herein an improved system 100, method 200 and computer program product for storing audio and/or visual content of a user and an improved system 300, method 400 and computer program product for rendering audio and/or visual content of a user, which address the limitations associated with the existing techniques.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A system (100) for storing audio and/or visual content of a user (500), the system (100) comprising one or more processors (102) configured to:
in response to a detection of a gesture (502) and/or voice command (504) of a user (500), identify a location (606) on a body of a subject (600) indicated by the gesture (502) and/or voice command (504); and
control at least one memory (104) to store an indication of the identified location (606) on the body of the subject (600) with audio and/or visual content of the user (500) captured for a time period after or during which the gesture (502) and/or voice command (504) of the user (500) is detected.

2. The system (100) as claimed in claim 1, wherein the system (100) comprises one or more detectors (106) configured to:
detect the gesture (502) and/or voice command (504) of the user (500); and/or
capture the audio and/or visual content of the user (500).

3. The system (100) as claimed in any of the preceding claims, wherein:
the time period has a predefined duration; or
the time period ends in response to a detection of a further gesture and/or voice command of the user (500).

4. The system (100) as claimed in any of the preceding claims, wherein the gesture (502) comprises a pointing gesture, a tapping gesture, a sliding gesture and/or an eye gesture.

5. The system (100) as claimed in any of the preceding claims, wherein the gesture (502) comprises:
a gesture of a body part of the user (500); or
a gesture of a device held by the user.

6. The system (100) as claimed in any of the preceding claims, wherein the one or more processors (102) are configured to identify the location (606) on the body of the subject (600) by being configured to:
process visual content of the user (500) captured for the time period during which the gesture (502) and/or voice command (504) of the user (500) is detected, using body recognition to identify the location (606) on the body of the subject (600) indicated by the gesture (502) and/or voice command (504); and/or
use body-coupled communication to identify the location (606) on the body of the subject (600) indicated by the gesture (502) and/or voice command (504).

7. A method (200) for storing audio and/or visual content of a user, the method (200) comprising:
in response to a detection of a gesture and/or voice command of a user, identifying (202) a location on a body of a subject indicated by the gesture and/or voice command; and
controlling (204) at least one memory to store an indication of the identified location on the body of the subject with audio and/or visual content of the user captured for a time period after or during which the gesture and/or voice command of the user is detected.

8. A system (300) for rendering audio and/or visual content of a user (500), the system (300) comprising one or more processors (302) configured to:
in response to a detection of a gesture (602) and/or voice command (604) of a subject (600), identify the subject (600) and a location (606) on a body of the subject (600) indicated by the gesture (602) and/or voice command (604);
acquire, from at least one a memory (304), audio and/or visual content of a user (500) stored with an indication of the identified location (606) on the body of the identified subject (600); and
control one or more user interfaces (308) to render the acquired audio and/or visual content of the user (500).

9. The system (300) as claimed in claim 8, wherein the system (300) comprises:
one or more detectors (306) configured to detect the gesture (602) and/or voice command (604) of the subject (600); and/or
one or more user interfaces (308) configured to render the acquired audio and/or visual content of the user (500).

10. The system (300) as claimed in any of claims 8 to 9, wherein one or more processors (302) are configured to:
control one or more user interfaces (308) to render a visual representation of the subject (600) indicating one or more locations (606) on the body of the subject (600) for which an indication is stored in the at least one memory (304); and/or
control one or more user interfaces (308) to render feedback indicative of a proximity of the gesture (602) to a location (606) on the body of the subject (600) for which an indication is stored in the at least one memory (304).

11. The system (300) as claimed in any of claims 8 to 10, wherein one or more processors (302) are configured to:
control one or more user interfaces (308) to render the acquired audio and/or visual content of the user (500) with a visual representation of the body of the identified subject (600).

12. The system (300) as claimed in any of claims 8 to 11, wherein one or more processors (302) are configured to identify the subject (600) by being configured to:
process visual content of the subject (600) captured for a time period during which the gesture (602) and/or voice command (604) of the subject (600) is detected, using camera-based recognition to identify the subject (600);
process audio content of the subject (600) captured for the time period during which the gesture (602) and/or voice command (604) of the subject (600) is detected, using voice recognition to identify the subject (600); and/or
use body-coupled communication to identify the subject (600).

13. The system (300) as claimed in any of claims 8 to 12, wherein one or more processors (302) are configured to identify the location (606) on the body of the subject (600) by being configured to:
process visual content of the subject (600) captured for a time period during which the gesture (602) and/or voice command (604) of the subject (600) is detected, using body recognition to identify the location (606) on the body of the subject (600) indicated by the gesture (602) and/or voice command (604); and/or
use body-coupled communication to identify the location (606) on the body of the subject (600) indicated by the gesture (602) and/or voice command (604).

14. A method (400) for rendering audio and/or visual content of a user, the method (400) comprising:
in response to a detection of a gesture and/or voice command of a subject, identifying (402) the subject and a location on a body of the subject indicated by the gesture and/or voice command;
acquiring (404), from at least one memory, audio and/or visual content of a user stored with an indication of the identified location on the body of the identified subject; and
controlling (406) one or more user interfaces to render the acquired audio and/or visual content of the user.

15. A computer program product comprising a computer readable medium, the computer readable medium having computer readable code embodied therein, the computer readable code being configured such that, on execution by a suitable computer or processor, the computer or processor is caused to perform the method as claimed in claim 7 or 14.
